Europäisches Patentamt

European Patent Office

Office européen des brevets

(11) Publication number: **0 062 477**
**B1**

(12) **EUROPEAN PATENT SPECIFICATION**

(45) Date of publication of patent specification: **12.02.86**

(51) Int. Cl.⁴: **G 10 K 11/26,** G 10 K 11/34

(21) Application number: **82301651.4**

(22) Date of filing: **30.03.82**

(54) Ultrasonic measuring apparatus.

(30) Priority: **31.03.81 JP 48275/81**

(43) Date of publication of application:
**13.10.82 Bulletin 82/41**

(45) Publication of the grant of the patent:
**12.02.86 Bulletin 86/07**

(84) Designated Contracting States:
**DE FR GB NL SE**

(56) References cited:
**EP-A-0 022 966
DE-A-3 008 553
FR-A-2 302 656
US-A-3 457 543**

**IBM TECHNICAL DISCLOSURE BULLETIN, vol.
22, no. 4, September 1979, pages 1696-1697;
New York, US REID AND WANG: "Computer-
Controlled Acoustic Beam steering and
Focusing with Variable Frequency Array
Transducers"**

(73) Proprietor: **FUJITSU LIMITED
1015, Kamikodanaka Nakahara-ku
Kawasaki-shi Kanagawa 211 (JP)**

(72) Inventor: **Miwa, Hirohide
6-7-10, Miyazaki Takatsu-ku
Kawasaki-shi Kanagawa, 213 (JP)**
Inventor: **Hayasahi, Hajime
8-7-21, Tsukimino
Yamato-shi Kanagawa, 242 (JP)**
Inventor: **Shimura, takaki
29-44, Tsurukawa 4-chome
Machida-shi Tokyo, 104-01 (JP)**

(74) Representative: **Sunderland, James Harry et al
HASELTINE LAKE & CO Hazlitt House
28 Southampton Buildings Chancery Lane
London WC2A 1AT (GB)**

Courier Press, Leamington Spa, England.

**Description**

The present invention relates to ultrasonic measuring apparatus.

Ultrasonic measuring instruments of the type utilizing transmission, reflection and scattering of ultrasonic waves, such as an ultrasonic camera, a hologram device, an echo-imaging device, a computed tomograph device, an aperture synthesizer, etc., employ a continuous wave or a pulsed wave of a predetermined frequency (or frequency band). An ultrasonic transducer for use in such measuring instruments is constituted by a single ultrasonic transducer element or by a plurality of ultrasonic transducer elements (assembled, for instance, in the form of an annular array, a linear scan array, a sector scan array, a discrete aperture synthesizing array, or the like). In the latter case, ultrasonic transducer elements are driven simultaneously or selectively and, in some cases, transmitting and receiving timings of the elements are varied.

For instance, when measurements are being made in relation to a living body, it is known that reflection and attenuation of an ultrasonic wave are subject to frequency dependencies which correspond to the type of living tissue encountered. Accordingly, by applying ultrasonic waves of a plurality of frequencies to the living body and detecting reflected waves or transmitted waves therefrom corresponding to the applied frequencies, more information can be obtained than in a case in which ultrasonic waves of a single frequency are used. In a case in which a plurality of frequencies are employed it is necessary that acoustic fields corresponding to the frequencies be of the same shape.

The acoustic field provided by transmission from an ultrasonic transducer is substantially dependent on the frequency used and the aperture configuration of the transducer; therefore, the acoustic field provided by transmission from the transducer differs for different frequencies when employing ultrasonic waves of a plurality of frequencies.

DE—A—3 008 553 discloses a system with a transducer assembly which simultaneously transmits ultrasonic beams of a plurality of frequencies with a reduced aperture diameter for a higher frequency beam to bring the shapes of the beams into agreement far from the transducer assembly.

According to the present invention there is provided ultrasonic measuring apparatus having a probe operable to transmit or to receive ultrasonic waves of a plurality of different frequencies simultaneously, wherein means are provided for selectively changing the size of a transmission or reception aperture of the probe for ultrasonic waves of at least one of the different frequencies to make the shape of an effective acoustic field of ultrasonic waves of that one frequency change selectively so as to substantially coincide with the shape of an effective acoustic field of ultrasonic waves of another of the different frequencies in respective different selected distance-from-the-probe ranges.

An embodiment of the present invention provides ultrasonic measuring apparatus in which a plurality of ultrasonic transducer elements are selectively driven in accordance with different desired distance ranges from an ultrasonic transducer so as to make the shapes of effective acoustic fields of ultrasonic waves of a plurality of frequencies substantially identical in the different desired distance ranges. This provides for enhanced accuracy in analysis of tissue or the like, the subject of measurement, having frequency dependent effects upon ultrasonic waves.

An embodiment of the present invention provides ultrasonic measuring apparatus in which a plurality of ultrasonic transducer elements are driven with driving waveforms based on their frequency dependencies.

Briefly stated, an ultrasonic measuring apparatus embodying the present invention is provided with a probe made up of a plurality of ultrasonic transducer elements and is adapted for simultaneous transmission or reception of ultrasonic waves of a plurality of frequencies. Means are provided for changing the shapes of the effective acoustic fields of the ultrasonic waves of different frequencies and are controlled so that the effective acoustic fields can assume substantially the same shape in different distance—from the probe ranges.

The present invention is concerned with the application of ultrasonic waves to a subject of measurement and the detection of transmitted or reflected wave from the inside of the subject. More particularly, the present invention is concerned with ultrasonic measurement involving the transmission of ultrasonic waves of a plurality of frequencies from an ultrasonic transducer and provides for control of respective effective acoustic fields of the ultrasonic waves so that they can be made substantially the same within each of different selected distance ranges from the ultrasonic transducer.

Reference is made, by way of example, to the accompanying drawings, in which:

Figure 1A is a schematic diagram explanatory of the acoustic field of an ultrasonic beam from a single transducer;

Figure 1B is a schematic diagram explanatory of an example of a probe in accordance with an embodiment of the present invention and ultrasonic beam production thereby,

Figures 2A and 2B are, respectively, a front-end-on view and a sectional view of another example of a probe employed in an embodiment of the present invention;

Figures 3A and 3B are schematic diagrams explanatory of far and near acoustic fields respectively which can be provided by the probe shown in Figures 2A and 2B;

Figures 4A, 4C and 4D are block diagrams illustrating principal parts of respective drive circuits of embodiments of the present invention;

Figure 4B illustrates parts of a drive circuit which can be employed in an embodiment of the present invention;

Figures 5A and 5D are graphs for assistance in explanation of drive signal waveforms and the control of a main lobe and side lobes in dependence thereupon,

Figures 6A to 6C are graphs respectively illustrating frequency vs. gain characteristic curves for transducer elements, frequency vs. gain characteristic curves of drive circuits, and combined frequency vs. power characteristic curves, for assistance in explanation of an embodiment of the present invention;

Figures 7A and 7B are schematic diagrams explanatory of linear and sector scan array type probes, respectively; and

Figure 8 is a schematic diagram explanatory of the principle of an embodiment of the present invention using mechanical switching of the shapes of effective acoustic fields.

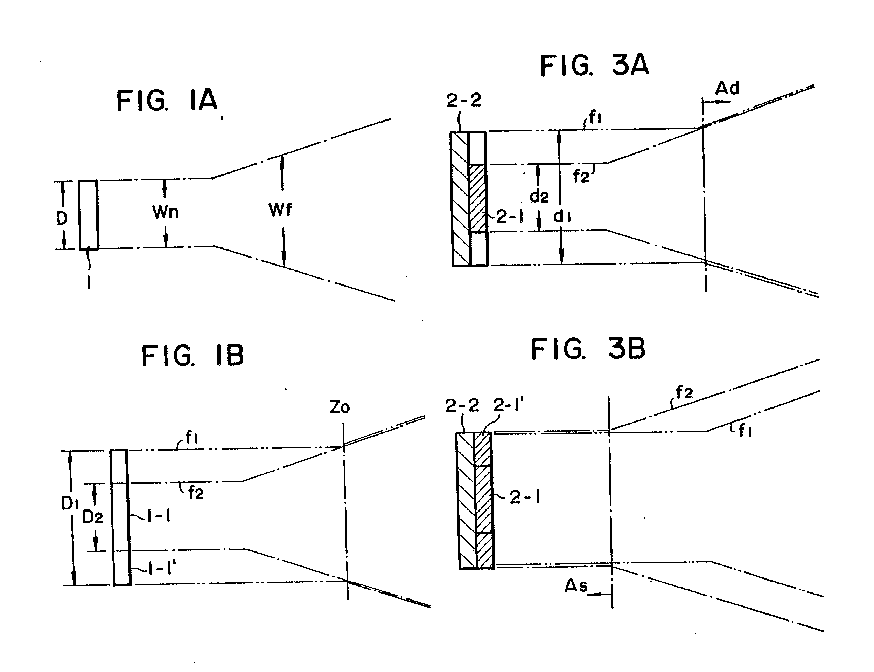

As illustrated in Figure 1A, in a near field part of the acoustic field of a disc-shaped ultrasonic transducer 1 having a diameter D, which near field part corresponds to

$$Z \leqq 1.6 \frac{D^2}{4\lambda} \qquad (1)$$

where $\lambda$ is the wavelength of an ultrasonic wave transmitted from the ultrasonic transducer 1 and Z is the distance therefrom, the diameter $W_n$ of the ultrasonic beam is given by

$$W_n \fallingdotseq D \qquad (2)$$

In a far field part of the acoustic field of the transducer, which far field part corresponds to

$$Z > 1.6 \frac{D^2}{4\lambda} \qquad (3)$$

the diameter $W_f$ of the ultrasonic beam is given by

$$W_f = 2.5 \frac{\lambda \cdot Z}{D} \qquad (4)$$

That is to say, the shape of the ultrasonic beam varies with frequency in the far field part.

In this specification the term 'effective acoustic field' is used as a general term for acoustic fields, for example such as are listed below.

(A) Transmitted acoustic field (actual acoustic field)

This is an acoustic field produced by the actually transmitted ultrasonic waves from a transducer, and is an acoustic field actually produced in the subject of measurement by (i) a diverging wave in an acousto-optic camera, a hologram device or aperture synthesis device (ii) a beam-focused wave of a plain or concave transducer, or (iii) focusing or deflection by timing control of an array element.

(B) Acoustic field to be received (receiving sensitivity field)

This is a field of sensitivity spatial distribution in the case of receiving, by an ultrasonic transducer, an ultrasonic wave transmitted from a certain point in the subject of measurement by virtue of transmission, refraction, reflection or scattering. That is, this field corresponds to the spatial distribution of sensitivity of a receiving transducer for ultrasonic waves from points in the subject of measurement. This field is a receiving sensitivity field produced by or dependent upon (i) the directivity of a transducer, (ii) dynamic focussing in the receiving stage by selection or timing control of transducer elements forming the transducer, or (iii) deflection of sensitive direction.

(C) Trans-receive acoustic field (field for actually received signal)

The abovesaid acoustic field to be received (B) is in reality superimposed on the transmitted acoustic field actually produced in the subject of measurement to provide an actually received signal. Accordingly, the transreceive acoustic field is produced by superimposing spatially and temporally, the transmitted field and the receiving sensitivity field. In the case of a reflection method, the same transducer is in general used both for the transmission and for reception; in a transmission method, transducers disposed facing one another are used respectively for transmission and reception; whilst for an aperture synthesis device, an acousto-optical camer and a hologram device, a suitable trans-receive transducer arrangement is employed. In any case, however, the transmitted acoustic field and the acoustic field to be received are spatially and temporally superimposed on each other to yield an acoustic field of characteristics determined by both transmission and reception. The acoustic field is the trans-receive acoustic field.

(D) Signal processing acoustic field

Sometimes a received signal is subjected to a computing process. For instance, for a focused acoustic field a deconvolution calculation using an inverse spread function, or image processing, is sometimes effected. In aperture synthesis for a diverging acoustic field, the received signal is usually subjected to a computing process. By such processing, virtual focusing of the actually received signal is steepened or its spatial resolution is raised as if the acoustic field were steepened. The signal processing acoustic field is a virtual acoustic field which is obtained by such processing, or an acoustic field resulting from the processing.

Figure 1B is a schematic diagram explanatory of a probe and ultrasonic beam production in accordance with an embodiment of the present invention. The probe comprises a disc-shaped ultrasonic transducer element 1-1 and an annular ultrasonic transducer element 1-1'.

When simultaneously transmitting an ultrasonic wave of a frequency f2 from the disc-shaped ultrasonic transducer element 1-1 of a diameter D2 and an ultrasonic wave of a frequency f1 from both of the annular ultrasonic transducer element 1-1' of a diameter D1 and the disc-shaped transducer element 1-1, by making $D1 \cdot f1 = D2 \cdot f2$, the shapes of the transmitted ultrasonic beams can be made coincident (in accordance with Eqs (1) to (4)) with each other in a far field region at a distance greater than $Z_0$ from the ultrasonic transducer elements 1-1 and 1-1', as indicated by the single dot and double dot chain lines.

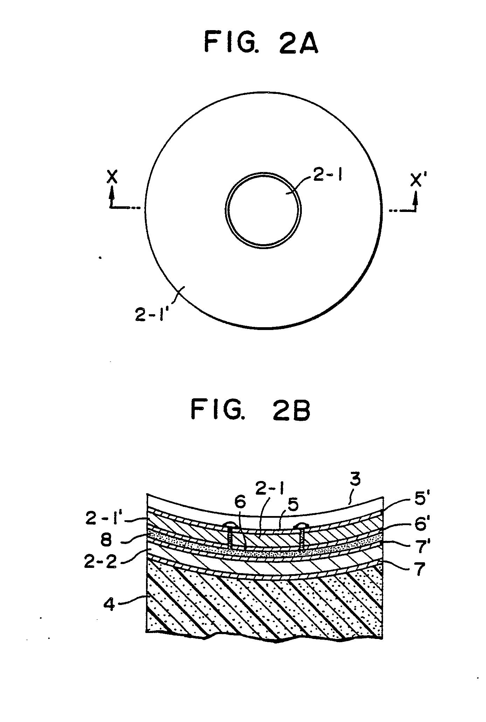

Figures 2A and 2B illustrate another example of a probe in accordance with an embodiment of the present invention, which probe comprises three ultrasonic transducer elements. Figure 2A is a front-end-on view of the probe and Figure 2B is a sectional view taken on the line X—X' in Figure 2A.

In Figures 2A and 2B, 2-1, 2-1' and 2-2 are ultrasonic transducer elements made of piezoelectric elements commonly referred to as PZT or PVDF; 3 is an acoustic impedance matching layer; 4 is a backing absorption layer; 5, 5', 6, 6', 7 and 7' are electrodes; and 8 represents electrically insulating adhesives having a matching function.

The ultrasonic transducer element 2-1 is disc-shaped, the transducer element 2-1' is annular is shape and disposed outside the transducer element 2-1, and the element 2-2 is disc shaped and of substantially the same diameter as the transducer element 2-1'. The transducer elements are concave in form. The electrodes 5 and 5' of the transducer elements 2-1 and 2-1' are interconnected and grounded. The electrodes 6 and 6' are separated from each other and each has connected thereto a lead wire (not shown). Accordingly, the transducer elements 2-1 and 2-1' can be selectively driven, by the selective application of drive signals to the electrodes 6 and 6'. Sandwiched between the electrodes 6, 6' and 7, 7' are electrically insulating adhesives. By applying a drive signal across the electrodes 7 and 7', the transducer element 2-2 is driven.

. Human tissue, as the subject of measurement, has an acoustic impedance of about 1.5 (Kg/m²S), PZT has an impedance of about 35 (Kg/m²S) and PVDF has an impedance of approximately 4 (Kg/m²S). The materials and thicknesses of the acoustic impedance matching layer 3 and the electrically insulating adhesives 8 are selected, as are acoustic impedances of the front transducer elements 2-1 and 2-1' and the rear transducer element 2-2, to ensure matching of acoustic impedances. It is advantageous, for example, to constitute the front transducer elements 2-1 and 2-1', which face or confront human tissue in use, by PVDF elements and the rear transducer element 2-2 by a PZT element. The backing absorption layer 4 absorbs ultrasonic waves emitted backwards and is made, for instance, of a mixture of epoxy resin with tungsten powder.

In a case in which the transducer elements 2-1 and 2-1' are of PVDF and the transducer element 2-2 is of PZT and ultrasonic waves of center frequencies f1 and f2 (f1<f2) and band widths ±Δf1 and ±Δf2, respectively, are to be transmitted, the thickness of the transducer element 2-2 is selected to be 1/2 of a wavelength λ1 corresponding to the frequency f1, and the material and thickness of the electrically insulating adhesives 8 are selected so that ultrasonic waves of a band width ranging from a frequency (f1−Δf1) to (f1+Δf1) may be transmitted from the transducer element 2-2. The effective thicknesses of the transducer elements 2-1 and 2-1' (an integrated value arrived at in dependence upon the effects of the acoustic impedance matching layer 3, the transducer elements 2-1 and 2-1' and the electrically insulating adhesives 8) are selected to be 1/4 of a wavelength λ2 corresponding to the frequency f2, and the material and thickness of the acoustic impedance matching layer 3 overlying the transducer elements 2-1 and 2-1' are selected so that the transducer elements 2-1 and 2-1' may transmit ultrasonic waves of a band width from a frequency (f2−Δf2) to (f2+Δf2). The ratio between the diameter d1 of the transducer element 2-1 and the outer diameter d2 of the transducer element 2-1' is selected as follows:

$$\frac{d1}{d2} = \frac{\lambda 1}{\lambda 2} \qquad (4)$$

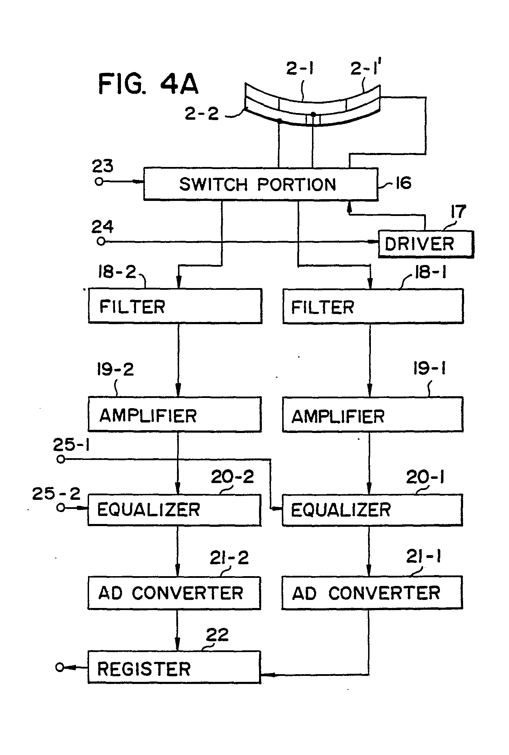

Figures 3A and 3B are for assistance in explaining how the shapes of acoustic fields of ultrasonic waves can be made coincident with one another through the use of the probe shown in Figures 2A and 2B.

When the shapes of far fields Ad are to be made to coincide with one another the transducer elements 2-1 and 2-2 are driven as indicated in Figure 3A. That is, ultrasonic waves of frequencies f2 and f1 are transmitted from the transducer elements 2-1 and 2-2, respectively (as indicated by shading lines). Through fulfilment of the condition of Eq. (4) (by selection of d1 and d2 so that in the far field $W_f$ as given by equation (4) is the same for both ultrasonic beams for each value of Z), the ultrasonic beams of the frequencies f1 and f2 substantially coincide with each other in the far field region as indicated by the single dot and double dot chain lines.

As illustrated in Figure 3B, by transmitting ultrasonic waves of frequency f2 from the transducer elements 2-1 and 2-1' and ultrasonic waves of frequency f1 from the ultrasonic transducer element 2-2, the shapes of near fields As are made to coincide with one another since the aperture diameters for emitting the ultrasonic waves of the frequencies f1 and f2 are the same.

As described above, by selecting the ultrasonic transducer element 2-1, driven at the frequency f2, in combination with the ultrasonic transducer element 2-2, driven at the frequency f1, the shapes of the far fields can be made substantially the same and, by selecting the ultrasonic transducer elements 2-1 and 2-1', in combination with the transducer element 2-2, the shapes of the near fields can be rendered almost identical with each other.

Figure 4A illustrates in block form apparatus embodying the present invention in which the probe shown in Figures 2A and 2B is used both for transmitting and receiving ultrasonic waves. The transducer elements 2-1, 2-1' and 2-2 are only schematically shown in Figure 4A for the clarity of illustration.

In Figure 4A, 16 is a switch portion; 17 is a drive; 18-1 and 18-2 are filters; 19-1 and 19-2 are amplifiers; 20-1 and 20-2 are equalizers; 21-1 and 21-2 are AD converters; 22 is a register; and 23, 24, 25-1 and 25-2 are control signal input terminals.

The driver 17 operates on a control signal from the input terminal 24 and yields, for instance, an impulse which has a uniform signal component over the working frequency band. The switch portion 16 responds to a control signal from the input terminal 23 to perform switching control to determine whether the output signal from the driver 17 should be applied to all of the transducer elements 2-1, 2-1' and 2-2 or only to transducer elements 2-1 and 2-2. At the same time, the switch portion 16 operates to supply the filters 18-1 and 18-2 with signals received by the transducer elements. In consequence, a signal received by the transducer element 2-2 is provided to the filter 18-2 and the signals or signal received by the transducer elements 2-1 and 2-1' or by the element 2-1 alone are or is fed to the filter 18-1.

A description will now be given of the measuring operations.

(1) Measurement of near acoustic field

The switch portion 16 operates on the control signal to apply the output signal from the driver 17 to the transducer elements 2-1, 2-1' and 2-2. The transducer elements 2-1 and 2-1' together transmit an ultrasonic beam of center frequency f2 and the transducer element 2-2 transmits an ultrasonic beam of center frequency f1. Therefore, the shapes of the acoustic fields produced by the ultrasonic waves of the frequencies f1 and f2 coincide with one another in the near field as described previously with reference to Figure 3B.

Of reflected waves from the near acoustic field in the subject of measurement, an ultrasonic wave of center frequency f1 is converted by the transducer element 2-2 into an electric signal, and an ultrasonic wave of center frequency f2 is converted by the transducer elements 2-1 and 2-1' into an electric signal. The former and latter electric signals thus obtained are respectively delivered to the filters of center frequencies f2 and f1 for removal of unnecessary components, thereafter being applied to the amplifiers 19-1 and 19-2 respectively.

The output signals from the amplifiers 19-1 and 19-2 are respectively provided to the equalizers 20-1 and 20-2, wherein they are subjected to correction of attenuation while transmission in the subject of measurement (correction of attenuation resulting from passage through the subject of measurement) and so forth in accordance with control signals from the input terminals 25-1 and 25-2. The equalizer outputs are converted by the AD converters 21-1 and 21-2 into digital signals, which are set in the register 22. The contents of the register 22 are sent to a processor or the like for performing processing such as analysis of the subject of the measurement, image display or the like.

(2) Measurement of far acoustic field

The switch portion 16 operates on the control signal to apply the output signal from the driver 17 to the transducer elements 2-1 and 2-2. In consequence, the transducer element 2-1 transmits an ultrasonic beam of center frequency f2 and the transducer element 2-2 transmits an ultrasonic beam of the center frequency f1. As described previously with reference to Figure 3A, the shapes of the acoustic fields provided by the ultrasonic waves of the frequencies f1 and f2 coincide with one another in the far field. Further, the switch portion 16 operates to supply the filter 18-1 with a signal of center frequency f2 received by the transducer element 2-1 and the filter 18-2 with a signal of center frequency f1 received by the transducer element 2-2. The equalizers 20-1 and 20-2 and other devices operate in the same manner as in the case of the measurement in relation to the near acoustic field described above, and measured digital values are set in the register 22. In the contents of the register 22 are sent to the processor or the like.

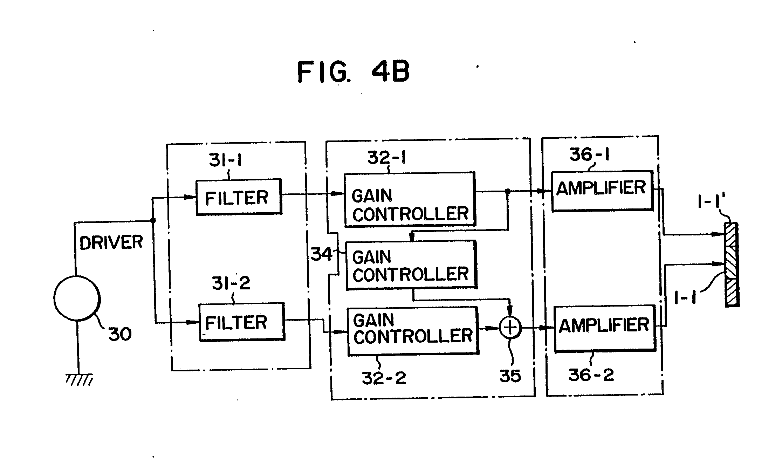

Figure 4B is a block diagram illustrating parts of a drive circuit which can be employed in an embodiment of the present invention.

Figure 4B shows an arrangement for use in driving the probe described previously in connection with Figure 1B, in which the annular ultrasonic transducer element 1-1' is disposed around the outer periphery of the disc-shaped ultrasonic transducer element 1-1.

In operation of the arrangement of Figure 4B, a driver 30 provides a signal having a component over a wide band width, which signal is delivered to a filter 31-1 of center frequency f1 and a filter 31-2 of center frequency f2. Output signals from the filters 31-1 and 31-2 are adjusted by gain controllers 32-1 and 32-2 to desired magnitudes, and the signal of center frequency f1 is amplified by an amplifier 36-1 and then fed to the transducer element 1-1'.

A portion of the output signal from the gain controller 32-1 is delivered via a gain controller 34 to an adder 35, wherein it is added to the output signal from the gain controller 32-2. The adder output is applied to the transducer element 1-1 after being amplified by an amplifier 36-2. Consequently, the transducer element 1-1 is driven by the signals of center frequencies f1 and f2.

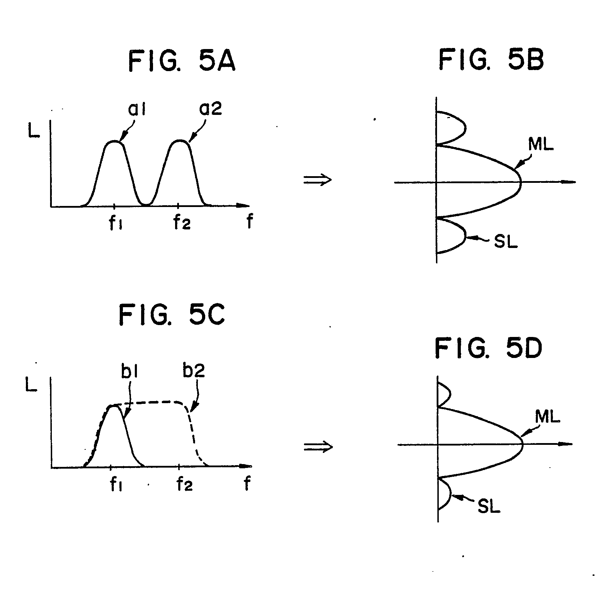

The reason why transducer element 1-1 is driven by signals of centre frequencies f1 and f2 will be explained with reference to Figures 5A to 5D. Figures 5A and 5C are graphs indicating relationships between driving signal level L and frequency f.

Figure 5A illustrates a case in which driving signals a1 and a2 of center frequencies f1 and f2 are applied to the transducer elements 1-1' and 1-1, respectively. In this case the shapes of the acoustic fields of the ultrasonic waves of the frequencies f1 and f2 coincide with each other at a position more than the distance $Z_O$ from the probe, as indicated previously with reference to Figure 1B, but in this case a main lobe ML is accompanied by relatively large side lobes as depicted in Figure 5B. By applying a signal b1 of the center frequency f1 and a signal b2 including the frequencies f1 and f2, as shown in the graph of Figure 5C, to the transducer elements 1-1' and 1-1, respectively, the side lobes SL can be made small relative to the main lobe, as illustrated in Figure 5D.

In Figure 4B, since a signal including signals of center frequencies f1 and f2 can be obtained from the adder 35, the side lobes can be reduced by driving the transducer element 1-1 with a signal b2 as shown in Figure 5C.

In practice, it is difficult to fabricate a transducer which has the same characteristics for a plurality of frequencies f1 and f2. Especially in a case in which the centre frequencies f1 and f2 are far apart there usually result frequency-gain characteristics as indicated by curves $H_1(\omega)$ and $H_2(\omega)$ in the graph of Figure 6A. For instance, the gain G of the transducer element 1-1' of center frequency f1 has a frequency characteristic as indicated by curve $H_1(\omega)$ and the gain G of the transducer element 1-1 of center frequency f2 has a frequency characteristic as indicated by curve $H_2(\omega)$.

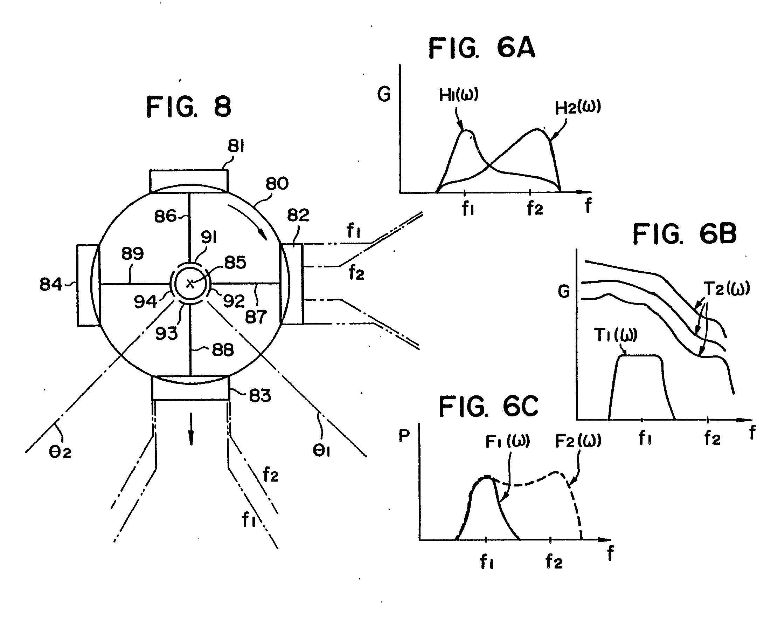

To deal with this the frequency-gain characteristic of the drive circuit is controlled as shown in the graph of Figure 6B. That is to say, the gains G of the transducer elements 1-1' and 1-1 are adjusted so as to have characteristics as indicated by $T_1(\omega)$ and $T_2(\omega)$ in Figure 6B respectively. Although in Figure 6B it is indicated by the three curves $T_2(\omega)$ that the gain can be adjusted to give three kinds of characteristics, the drive circuit is not limited specifically to providing one or any of these characteristics.

By combination of the frequency-gain characteristics shown in the graphs of Figures 6A and 6B, the powers P of the ultrasonic waves transmitted become, for example, as illustrated in the graph of Figure 6C. Namely, by providing power versus frequency characteristics, respectively as indicated by $F_1(\omega)$ and $F_2(\omega)$ in Figure 6C, for ultrasonic waves transmitted from the transducer elements 1-1' and 1-1, it is possible to markedly increase the side lobes and obtain a favourable main lobe configuration.

The frequency-gain characteristic of the drive circuit such as described above can be adjusted by the gain controllers 32-1, 32-2 and 34 in Figure 4B.

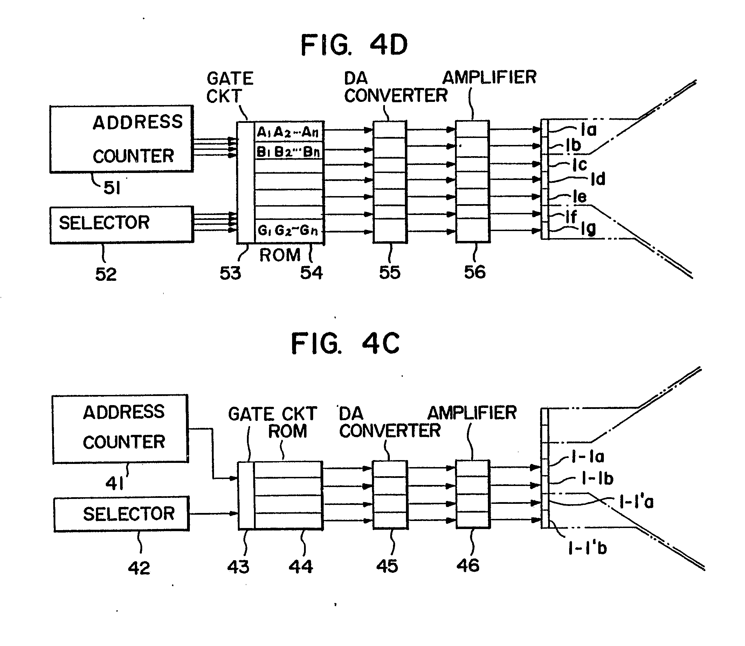

Figure 4C is a block diagram illustrating an embodiment of the present invention using a probe in which the transducer elements 1-1 and 1-1' of Figure 1B are each subdivided into concentric elements, 1-1a, 1-1b and 1-1'a, 1-1'b, respectively. Figure 4C shows the drive circuit for this embodiment of the present invention.

The drive circuit of Figure 4C has a memory 44, such as a read only memory or the like, which has stored therein, in correspondence to the individual transducer elements 1-1a, 1-1b, 1-1'a and 1-1'b, data indicating driving signal waveforms which provide such frequency-gain characteristics as are described with reference to Figures 6A, 6B and 6C. Furthermore, the data is selected so that the transducer elements can be driven in a manner providing a higher intensity in the central portion of the ultrasonic beam than in the peripheral portion thereof, as is the case with a Gaussian distribution.

An address counter 41 counts clock pulses to generate an address signal for the memory 44, and the address signal is applied via a gate circuit 43 to the memory 44. A selector 42 controls the gate circuit 43 to select the driving signal waveform data stored in the memory 44 and to selectively drive the transducer elements. Data sequentially read out from the memory 44 by stepping of the address signal is converted by a DA converter 45 into analog signals, which are amplified by an amplifier 46 and fed to the transducer elements 1-1a, 1-1b, 1-1'a and 1-1'b to drive them. For instance, the transducer elements 1-1a and 1-1b are driven by a drive signal including frequencies f1 and f2, and the elements 1-1' and 1-1'b are driven by a drive signal of frequency f1.

Figure 4D illustrates in block form an embodiment of the present invention having a probe constituted by a linear array transducer. Ultrasonic transducer elements 1a to 1g are arranged in a line, and data indicating driving signal waveforms corresponding to the transducer elements is stored in a memory 54 of the driving circuit of the embodiment. By the application of a sequentially stepping address signal from an address counter 51 via a gate circuit 53 to the memory 54, data $(A_1, B_1, \ldots G_1)$ to $A_n, B_n, \ldots G_n)$ corresponding to the individual transducer elements is sequentially read out from the memory 54 and converted by a DA converter 55 into analog signals, which are amplified by an amplifier 56 and then delivered to the transducer elements

By applying a driving signal including frequencies f1 and f2 to the transducer elements 1c to 1e and a driving signal of frequency f1 to the transducer elements 1a, 1b, 1f and 1g, the shapes of acoustic fields of the ultrasonic waves of the frequencies f1 and f2 can be made to coincide with each other at a predetermined distance from the probe. Moreover, the shapes of the acoustic fields can be changed by selectively driving the

transducer elements 1a to 1g under the control of a selector 52. Accordingly, it is possible to carry out switching between measurement in the near acoustic field and measurement in the far acoustic field as described previously in connection with Figures 3A and 3B.

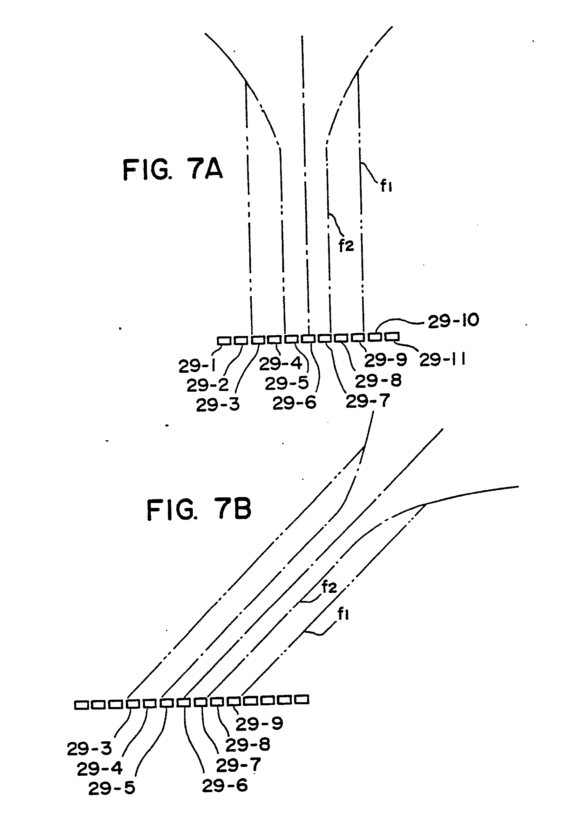

Figure 7A is explanatory of a linear scan array type probe, in which ultrasonic transducer elements 29-1 to 29-11 are aligned and each element is adapted to be capable of transmitting ultrasonic waves including the frequencies f1 and f2. In this case, each transducer element can be constructed so that it is able to transmit an ultrasonic wave including the frequencies f1 and f2 by appropriate selection of the driving signal waveform, for example, as in the case of the transducer element 1-1 in Figure 4B, or by laminating together a transducer element 2-2 of operating frequency f1 and transducer elements 2-1 and 2-1' of operating frequency f2 as depicted in Figures 2A and 2B.

In consequence, during measurement in the near acoustic field, ultrasonic waves including the frequencies f1 and f2 are transmitted from each of the transducers 29-3 and 29-9. During measurement in the far acoustic field ultrasonic waves of frequency f1 are transmitted from each of the transducer elements 29-3, 29-4, 29-8 and 29-9 and ultrasonic waves including the frequencies f1 and f2 are transmitted from each of the transducer elements 29-5 to 29-7, whereby the shapes of the far acoustic fields can be made to coincide with each other as indicated by the single-dot and double-dot chain lines.

Figure 7B is explanatory of a sector scan array type probe, in which ultrasonic beams can be directed obliquely aslant by sequentially displacing the phases of driving signals that are applied to the transducer elements 29-3 to 29-9. Further, the direction of the ultrasonic beam can freely be varied by controlling the phases of the driving signals. In this case also, it is possible to perform switching between the measurements in the near and far acoustic fields by the selection of the transducer elements which transmit the ultrasonic waves includes the frequencies f1 and f2.

Figure 8 is explanatory of the principle of an arrangement for mechanically switching the shape of the effective acoustic field. On a probe head rotor 80, there are fixedly mounted transducers 81 to 84 which transmit ultrasonic waves the acoustic fields of which become uniform at a desired distance, and the probe head rotor 80 is arranged to be rotatable by a shaft 85. 86 and 89 are lead wires, and 91 to 94 are contactors. The contactors 91 to 94 make contact with a fixed contact not shown. For example, when the contactor 93 contacts the fixed contact, the transducer 83 is driven.

In a case in which the transducer 82 is designed for measurement in the far acoustic field and the transducer 83 for measurement in the near acoustic field, the near acoustic field can be measured when the probe head rotor 80 held at the position illustrated in Figure 8. By turning the probe head rotor 80 through 90° in the direction indicated by the arrow in Figure 8, the far acoustic field can be measured by the transducer 82 which assumes a position in an angular range between θ1 and θ2. In other words, the shapes of the acoustic fields of the ultrasonic waves of the frequencies f1 and f2 can be altered by selectively switching the transducers. Incidentally, the shaft 85 can be driven by any desired drive mechanism, such as a motor or the like. The number of the transducers 81 to 84 mounted on the probe rotor head 80 can also be decreased or increased. Moreover, the connection of the transducers 81 to 84 with the stationary part of the mechanism can be achieved by means of transformer coupling. The probe head rotor 80 and the transducers 81 to 84 are housed in a vessel filled with oil, jelly or like fluids so as to prevent the transducers from making direct contact with the subject of measurement when the probe head rotor 80 is turning.

The shape of the effective acoustic field can be changed by electrically switching means providing for the selective driving of the ultrasonic transducer elements and for the selection of drive signals and can also be changed by mechanical switching means as shown in Figure 8. It is possible to employ suitable combinations of these two means. Near and far acoustic fields can be measured on a time-shared basis also. The number of the frequencies used is not limited to two frequencies (f1 and f2) but may be increased. For instance, in relation to Figure 4C, it is possible to drive the transducer element 1-1a at a frequency between f1 and f2, the element 1-1b at a frequency between f1 and f2' (f2'<f2), the element 1-1'a at a frequency between f1 and f1' (f1'>f1) and the element 1-1'b at the frequency f1.

Further, embodiments of the present invention are not limited specifically to two-stage switching between measurements in the near and far acoustic fields. Embodiments of this invention can provide multistage switching between different ranges of coincidence of the shapes of acoustic fields (to provide shape coincidence in any selected one of a multiplicity of distance ranges) of a plurality of frequencies by selective driving of the transducer elements. The use of each an arrangement facilitates the analysis of a tissue moving in the body of the subject of measurement.

As has been described above, in an embodiment of the present invention, ultrasonic waves of a plurality of frequencies are simultaneously transmitted and the shapes of acoustic fields of the ultrasonic waves are made to coincide with each other at a desired distance range from a probe.

## Claims

1. Ultrasonic measuring apparatus, having a probe (1-1, 1-1'; Fig. 2A; Fig. 2B; 2-1, 2-1', 2-2; 1-1a, 1-1b, 1-1'a, 1-1'b; 1a to 1g; 29-1 to 29-11; 80 to 94) operable to transmit or to receive ultrasonic waves of a plurality of different frequencies $(f_1, f_2)$

simultaneously, wherein means (16; 42; 52; 80 to 85) are provided for selectively changing the size ($D_1$, $D_2$, $d_1$, $d_2$) of a transmission or reception aperture of the probe for ultrasonic waves of at least one ($f_2$) of the different frequencies, to make the shape of an effective acoustic field of ultrasonic waves of that one frequency ($f_2$) change selectively so as to substantially coincide with the shape (Ad, As) of an effective acoustic field of ultrasonic waves of another of the different frequencies ($f_2$) in respective different selected distance-from-the-probe ranges.

2. Apparatus as claimed in claim 1, wherein the probe is made up of a plurality of ultrasonic transducer elements (1-1, 1-1'; 2-1, 2-1', 2-2; 1-1a, 1-1b, 1-1'a, 1-1'b; 1a to 1g; 29-1 to 29-11) which provide for the simultaneous transmission of or for the simultaneous reception of ultrasonic beams or waves respectively of the plurality of different frequencies ($f_1$, $f_2$).

3. Apparatus as claimed in claim 1 or 2, wherein the probe comprises a plurality of ultrasonic transducer elements (1-1, 1-1'; 2-1, 2-1'; 1-1a, 1-1b, 1-1'a, 1-1'b; 1a to 1g; 29-1 to 29-11) disposed one adjacent another, and wherein at least an ultrasonic transducer element (1-1; 2-1; 1-1a, 1-1b; 1c to 1d; 29-5 to 29-7) forming a central portion of the probe is capable of simultaneously transmitting or of simultaneously receiving ultrasonic beams of ultrasonic waves of different frequencies ($f_1$, $f_2$).

4. Apparatus as claimed in claim 1 or 2, wherein the probe comprises a plurality of ultrasonic transducer elements (2-1, 2-1', 2-2) of different resonant frequencies ($f_1$, $f_2$) laminated together, one of those transducer elements (2-1, 2-1') being itself divided into a further plurality of transducer elements, and wherein the apparatus is operable by selection in relation to the divided ultrasonic transducer element to change the size ($d_1$, $d_2$) of the transmission aperture of ultrasonic waves of a resonant frequency ($f_2$) transmitted from the divided ultrasonic transducer element.

5. Apparatus as claimed in any preceding claim, wherein the probe comprises a plurality of ultrasonic transducer elements (1a to 1g; 29-1 to 29-11) arranged in a linear array, each capable of simultaneously transmitting or of simultaneously receiving ultrasonic beams of waves of the said plurality of different frequencies ($f_1$, $f_2$), the probe being operable to carry out a linear or sector scan, and wherein the apparatus is operable to cause the size of a transmission or reception aperture of ultrasonic beams of waves of the said one ($f_2$) of the different frequencies to selectively change by selective operation of the transducer elements and by selection of the frequencies of ultrasonic waves transmitted or received by the transducer elements.

6. Apparatus as claimed in claim 1, wherein the probe comprises at least two different transducers (81 to 84), each transducer capable of simultaneously transmitting or of simultaneously receiving ultrasonic waves of a plurality of frequencies ($f_1$, $f_2$) and each transducer providing a different selected distance from-the-probe range in which the shapes of the effective acoustic fields of the ultrasonic waves of different frequencies substantially coincide with one another, and wherein the size changing means (80, 85) are operable to select different said transducers for employment thereby to change the size of the transmission or reception aperture of the probe for ultrasonic waves of the said one frequency ($f_2$).

7. Apparatus as claimed in claim 6, wherein the transducers (81 to 84) are mounted on a rotatable probe head rotor (80).

8. Apparatus as claimed in claim 2, 3 or 4, wherein each element of the plurality of ultrasonic transducer elements (1-1, 1-1'; 1-1a, 1-1b, 1-1'a, 1-1'b; 1a to 1g) is supplied with a driving signal of a level adjusted in accordance with the frequency-gain characteristic of the transducer element concerned.

9. Apparatus as claimed in claim 8, wherein waveform data, indicating waveforms of driving signals adjusted in accordance with the frequency-gain characteristics of the corresponding ultrasonic transducer elements, is stored in a memory (44, 54) in correspondence to the ultrasonic transducer elements (1-1a, 1-1b, 1-1'a, 1-1'b; 1a to 1g), and the driving signals are produced on the basis of the waveform data as sequentially read-out from the memory and are supplied to the ultrasonic transducer elements.

10. Use of apparatus as claimed in any preceding claim for carrying out measurements in relation to the interior of a subject of measurement by means of ultrasonic beams of ultrasonic waves of the plurality of different frequencies.

**Patentansprüche**

1. Ultraschallmeßgerät, mit einer Sonde (1-1, 1-1'; Fig. 2A; Fig. 2B; 2-1, 2-1', 2-2; 1-1a, 1-1b, 1-1'a, 1-1'b; 1a bis 1g; 29-1 bis 29-11; 80 bis 94), die im Betrieb Ultraschallwellen mit einer Vielzahl von verschiedenen Frequenzen ($f_1$, $f_2$) gleichzeitig übertragen oder empfangen kann, bei welchem Einrichtungen (16; 42; 52; 80 bis 85) vorgesehen sind, um selektiv die Größe ($D_1$, $D_2$, $d_1$, $d_2$) einer Übertragungs- oder Empfangsapertur der Sonde für Ultraschallwellen von wenigstens einer ($f_2$) der verschiedenen Frequenzen zu ändern, um die Form eines effektiven akustischen Feldes von Ultraschallwellen der einen Frequenz ($f_2$) selektiv zu ändern, um so im wesentlichen mit der Form (Ad, As) eines effektiven akustischen Feldes von Ultraschallwellen von einer anderen der verschiedenen Frequenzen ($f_2$) in entsprechend verschieden ausgewählten Bereichnen des Abstandes von der Sonde zu koinzidieren.

2. Gerät nach Anspruch 1, bei welchem die Sonde aus einer Vielzahl von Ultraschallwandlerelementen (1-1, 1-1'; 2-1, 2-1', 2-2; 1-1a, 1-1b, 1-1'a, 1-1'b; 1a bis 1g; 29-1 bis 29-11) besteht, welche für die gleichzeitige Übertragung von oder für den gleichzeitigen Empfang von Ultraschallstrahlen bzw. -wellen der Vielzahl von verschiedenen Frequenzen ($f_1$, $f_2$) sorgen.

3. Gerät nach Anspruch 1 oder 2, bei welchem die Sonde eine Vielzahl von Ultraschallwandlerelementen (1-1, 1-1'; 2-1, 2-1'- 1-1a, 1-1b, 1-1'a, 1-1'b; 1a bis 1g; 29-1 bis 29-11) umfaßt, die eines an das andere angrenzend angeordnet sind, und bei welchem wenigstens ein Ultraschallwandlerelement (1-1; 2-1; 1-1a, 1-1b; 1c bis 1d; 29-5 bis 29-7), das einen zentralen Teil der Sonde bildet, fähig ist, Ultraschallstrahlen oder Ultraschallwellen von verschiedenen Frequenzen ($f_1$, $f_2$) gleichzeitig zu senden oder gleichzeitig zu empfangen.

4. Vorrichtung nach Anspruch 1 oder 2, bei welcher die Sonde eine Vielzahl von Ultraschallwandlerelementen (2-1, 2-1', 2-2), von verschiedenen Resonanzfrequenzen ($f_1$, $f_2$), umfaßt, die miteinander laminiert sind, und ein von den Wandlerelementen (2-1, 2-1') selbst in eine weitere Vielzahl von Wandlerelementen unterteilt ist, und bei welchen das Gerät durch Auswahl in Relation und zu dem unterteilten Ultraschallwandlerelement betreibbar ist, um die Größe ($d_1$, $d_2$) der Übertragungsapertur von Ultraschallwellen von einer Resonanzfrequenz ($f_2$), die von dem unterteilten Ultraschallwandlerelement übertragen werden, zu ändern.

5. Vorrichtung nach einem der vorhergehenden Ansprüche, bei welcher die Sonde eine Vielzahl von Ultraschallwandlerelementen (1a bis 1g; 29-1 bis 29-11) umfaßt, die in einer linearen Anordnung angeordnet sind und jeweils Ultraschallstrahlen von Wellen der genannten Vielzahl von verschiedenen Frequenzen ($f_1$, $f_2$) gleichzeitig übertragen oder gleichzeitig empfangen können, die Sonde betätigbar ist, um eine lineare oder Sektorenabtastung auszuführen, und bei welchem das Gerät betreibbar ist, um zu verursachen, daß die Größe der Übertragungs- oder Empfangsapertur von Ultraschallstrahlen der Wellen der genannten einen ($f_2$) der verschiedenen Frequenzen wahlweise durch selektiven Betrieb der Wandlerelemente und durch Auswahl der Frequenzen von Ultraschallwellen, die von den Wandlerelementen übertragen oder empfangen werden, zu ändern.

6. Gerät nach Anspruch 1, bei welchem die Sonde wenigstens zwei verschiedene Wandler (81 bis 84) umfaßt, von denen jeder Wandler Ultraschallwellen von einer Vielzahl von Frequenzen ($f_1$, $f_2$) gleichzeitig übertragen oder gleichzeitig empfangen kann, und bei welchem jeder Wandler einen verschieden ausgewählten Abstand von dem Sondenbereich liefert, in welchem die Formen der effektiven akustischen Felder von den Ultraschallwellen im wesentlichen mit einer der verschiedenen Frequenzen koinzidieren, und bei welchem die Größenänderungseinrichtung (80, 85) betreibbar ist, um verschiedene der genannten Wandler zur Verwendung auszuwählen, um dadurch die Größe der Übertragungs- oder Empfangsapertur der Sonde für Ultraschallwellen der genannten einen Frequenz ($f_2$) zu ändern.

7. Gerät nach Anspruch 6, bei welchem die Wandler (81 bis 84) auf einem rotierbaren Sondenkopfrotor (80) montiert sind.

8. Gerät nach Anspruch 2, 3 oder 4, bei welchem jedem Element der Vielzahl von Ultraschallwandlerelementen (1-1, 1-1'; 1-1a, 1-1b, 1-1'a, 1-1'b; 1a bis 1g) ein Treibersignal mit einem Pegel zugeführt wird, welcher in Übereinstimmung mit der Frequenz-Verstärkungs-Charakteristik des betreffenden Wandlerelementes eingestellt ist.

9. Gerät nach Anspruch 8, bei welchem Wellenformdaten, die die Wellenformen der treibenden Signale anzeigen, die in Übereinstimmung mit den Frequenz-Verstärkungs-Charakteristiken der entsprechenden Ultraschallwandlerelemente eingestellt sind, in einem Speicher (44, 54) gespeichert werden, in Übereinstimmung mit den Ultraschallwandlerelementen (1-1a, 1-1b, 1-1'a, 1-1'b; 1a bis 1g), und die treibenden Signale auf der Basis der Wellenformendaten erzeugt werden, wie sie sequenziell von dem Speicher ausgelesen und den Ultraschallwandlerelementen zugeführt werden.

10. Verwendung eines Gerätes nach einem der vorhergehenden Ansprüche, um Messungen in Relation zu dem Inneren eines Gegenstands der Messung mit Hilfe von Ultraschallstrahlen von Ultraschallwellen von einer Vielzahl von verschiedenen Frequenzen durchzuführen.

**Revendications**

1. Dispositif de mesure à ultrasons, comportant une sonde (1-1, 1-1'; Fig. 2A; Fig. 2B; 2-1, 2-1', 2-2; 1-1a, 1-1b, 1-1'a, 1-1'b; 1a à 1g; 29-1 à 29-11; 80 à 94) agencée pour émettre et pour recevoir des ondes ultrasonores d'un ensemble de fréquences différentes ($f_1$, $f_2$) simultanément, caractérisé en ce qu'il comprend des moyens (16; 42; 52; 80 à 85) pour faire varier sélectivement la grandeur ($D_1$, $D_2$, $d_1$, $d_2$) d'une ouverture d'émission ou de réception de la sonde pour les ondes ultrasonores d'au moins une (f2) des fréquences différentes, pour que la forme d'un champ acoustique effectif d'ondes ultrasonores de ladite fréquence (f2) varie sélectivement de manière à ce qu'elle coïncide essentiellement avec la forme (Ad, As) d'un champ acoustique effectif d'ondes ultrasonores d'une autre des fréquences différentes (f2) dans des gammes différentes de distances de la sonde sélectionnées respectives.

2. Dispositif selon la revendication 1, caractérisé en ce que la sonde est constituée d'un ensemble d'éléments transducteurs à ultrasons (1-1, 1-1'; 2-1, 2-1', 2-2; 1-1a, 1-1b, 1-1'a, 1-1'b; 1a à 1g; 29-1 à 29-11) qui assurent l'émission simultanée ou la réception simultanée de faisceaux d'ultrasons ou d'ondes ultrasonores respectivement de l'ensemble des fréquences différentes ($f_1$, $f_2$).

3. Dispositif selon l'une quelconque des revendications 1 et 2, caractérisé en ce que la sonde comprend un ensemble d'éléments transducteurs à ultrasons (1-1, 1-1'; 2-1, 2-1'; 1-1a, 1-1b, 1-1'a, 1-1'b; 1a à 1g; 29-1 à 29-11) disposés les uns à côté des autres, et en ce qu'au moins un élément transducteur à ultrasons (1-1; 2-1; 1-1a, 1-1b; 1c à 1d; 29-5 à 29-7) constituant une partie centrale de la sonde est agencé pour émettre simultanément ou pour recevoir simultanément des faisceaux d'ultrasons d'ondes ultrasonores des fréquences différents (f₁, f₂).

4. Dispositif selon l'une quelconque des revendications 1 et 2, caractérisé en ce que la sonde comprend un ensemble d'éléments transducteurs à ultrasons (2-1, 2-1', 2-2) de différentes fréquences de résonance (f₁, f₂) qui sont stratifiés ensemble, un de ces éléments transducteurs (2-1, 2-1') étant lui-même divisé en un autre ensemble d'éléments transducteurs, et en ce que le dispositif est agencé pour fonctionner par sélection relative à l'élément transducteur à ultrasons divisé pour faire varier la grandeur (d₁, d₂) de l'ouverture d'émission des ondes ultrasonores d'une fréquence de résonance (f₂) émises par l'élément transducteur à ultrasons divisé.

5. Dispositif selon l'une quelconque des revendications 1 à 4, caractérisé en ce que la sonde comprend un ensemble d'éléments transducteurs à ultrasons (1a à 1g; 29-1 à 29-11) disposés en un réseau linéaire, chacun pouvant émettre simultanément ou recevoir simultanément des faisceaux d'ondes ultrasonores de l'ensemble des fréquence différentes (f₁, f₂), la sonde étant agencée pour effectuer un balayage linéaire ou de secteur, et en ce que le dispositif est agencé pour faire varier sélectivement la grandeur d'une ouverture d'émission ou de réception de faisceaux d'ondes ultrasonores de ladite fréquence (f₂) des fréquences différentes par un fonctionnement sélectif des éléments transducteurs et par sélection des fréquences des ondes ultrasonores émises ou reçues par les éléments transducteurs.

6. Dispositif selon la revendication 1, caractérisé en ce que la sonde comprend au moins deux transducteurs différents (81 à 84), chaque transducteur étant agencé pour émettre simultanément ou pour recevoir simultanément des ondes ultrasonores d'un ensemble de fréquences (f₁, f₂) et chaque transducteur fournissant une gamme différente de distances de la sonde sélectionnée dans laquelle les formes des champs acoustiques effectifs des ondes ultrasonores des fréquences différentes coïncident essentiellement entre elles, et en ce que les moyens pour faire varier la grandeur (80, 85) sont agencés pour sélectionner les différents transducteurs pour leur utilisation, la grandeur de l'ouverture d'émission ou de réception de la sonde pour des ondes ultrasonores de ladite fréquence (f₂) étant ainsi changée.

7. Dispositif selon la revendication 6, caractérisé en ce que les transducteurs (81 à 84) sont montés sur un rotor de tête de sonde (80) tournant.

8. Dispositif selon l'une quelconque des revendications 2, 3 et 4, caractérisé en ce que chaque élément de l'ensemble des éléments transducteurs à ultrasons (1-1, 1-1'; 1-1a, 1-1b, 1-1'a, 1-1'b; 1a à 1g) est agencé pour recevoir un signal de commande d'un niveau réglé conformément à la caractéristique de gain en fonction de la fréquence de l'élément transducteur concerné.

9. Dispositif selon la revendication 8, caractérisé en ce que des données de forme d'onde, indiquant les formes d'onde des signaux de commande réglés conformément au caractéristiques de gain en fonction de la fréquence des éléments transducteurs à ultrasons correspondants, sont mémorisées dans une mémoire (44, 54) en correspondance avec les éléments transducteurs à ultrasons (1-1a, 1-1b, 1-1'a, 1-1'b; 1a à 1g), et en ce que les signaux de commande sont produits sur la base des données de formes d'onde telles qu'elles sont lues séquentiellement dans la mémoire et sont envoyés aux éléments transducteurs à ultrasons.

10. Utilisation d'un dispositif selon l'une quelconque des revendications précédentes pour effectuer des mesures relatives à l'intérieur d'un objet de mesure au moyen des faisceaux d'ultrasons d'ondes ultrasonores de l'ensemble des fréquences différentes.

FIG. 3A

FIG. 3B

FIG. 1A

FIG. 1B

## FIG. 2A

## FIG. 2B

# FIG. 4A

SWITCH PORTION ~16

DRIVER 17

FILTER 18-2

FILTER 18-1

AMPLIFIER 19-2

AMPLIFIER 19-1

EQUALIZER 20-2

EQUALIZER 20-1

AD CONVERTER 21-2

AD CONVERTER 21-1

REGISTER 22

FIG. 4B

## FIG. 4D

ADDRESS COUNTER
51

SELECTOR
52

GATE CKT
53

ROM
54

| A₁ A₂··Aₙ |
| B₁ B₂···Bₙ |

| G₁ G₂~Gₙ |

DA CONVERTER
55

AMPLIFIER
56

Ia
Ib
Ic
Id
Ie
If
Ig

## FIG. 4C

ADDRESS COUNTER
41

SELECTOR
42

GATE CKT ROM
43    44

DA CONVERTER
45

AMPLIFIER
46

I-Ia
I-Ib
I-I'a
I-I'b

0 062 477

FIG. 5A

FIG. 5B

FIG. 5C

FIG. 5D

FIG. 8

FIG. 6A

FIG. 6B

FIG. 6C

FIG. 7A

f1

f2

29-10

29-1
29-2
29-3

29-4
29-5
29-6

29-7

29-8

29-9  29-11

FIG. 7B

f2

f1

29-3
29-4
29-5

29-6

29-7

29-8

29-9

8